(19) Europäisches Patentamt
European Patent Office
Office européen des brevets

(11) EP 1 586 306 A1

(12) **EUROPÄISCHE PATENTANMELDUNG**

(43) Veröffentlichungstag:
**19.10.2005 Patentblatt 2005/42**

(51) Int Cl.7: **A61K 7/42**

(21) Anmeldenummer: **05100879.5**

(22) Anmeldetag: **09.02.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **23.03.2004 DE 102004014614**

(71) Anmelder: **Beiersdorf AG**
**20245 Hamburg (DE)**

(72) Erfinder:
- **Batzer, Jan**
  **22459, Hamburg (DE)**
- **Doering, Thomas**
  **41542, Dormagen (DE)**
- **Sugar, Martin**
  **20257, Hamburg (DE)**
- **Wolber, Rainer**
  **22397, Hamburg (DE)**
- **Wendel, Volker**
  **60529 Frankfurt/Main (DE)**
- **Blatt, Thomas**
  **21379, Wedel (DE)**
- **Mundt, Claudia**
  **28207, Bremen (DE)**
- **Schulz, Jens**
  **22869, Schenefeld (DE)**
- **Dippe, Rixa**
  **20255, Hamburg (DE)**

(54) **Kosmetische und dermatologische Lichtschutzformulierungen**

(57) Kosmetische oder dermatologische Zubereitungen, dadurch gekennzeichnet, daß sie eine UVA-Balance von mehr als 15 aufweisen, und deren Verwendung.

EP 1 586 306 A1

## Beschreibung

Die vorliegende Erfindung betrifft kosmetische und dermatologische Zubereitungen mit einer hohen UVA-Balance sowie die Verwendung derartiger Zubereitungen zur Steigerung und/oder Verbesserung des natürlichen, hauteigenen UV-Schutzsystems, der Hautfeuchtigkeit und der Tiefenfeuchtigkeit der Haut, der Vitalität und des Schutzes von Hautzellen sowie die Verwendung zum Schutz de r Haut vor vorzeitiger Alterung und optisch wahrnehmbaren Veränderungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut:

Die so genannte UV-C-Strahlung mit einer Wellenlänge zwischen 100 und 280 nm wird von der Ozonschicht der Erdatmosphäre absorbiert und findet sich dementsprechend nicht im Sonnenspektrum. Sie ist daher ohne physiologische Bedeutung beim Sonnenbaden.

Der sogenannte UV-B-Bereich liegt zwischen 290 nm und 320 nm. UV-B-Strahlen sind wesentlich für die lang anhaltende Bräunung der Haut verantwortlich, können aber gleichzeitig ein Erythem, einen einfachen Sonnenbrand oder sogar mehr oder weniger starke Verbrennungen verursachen. Auch chronische Lichtschäden, Photodermatosen und Herpes solaris sowie Hautkrebs können durch UV-B-Strahlung hervorgerufen werden.

Da der Sonnenbrand ein typischer UV-B-Effekt ist, enthalten viele kosmetische oder dermatologische Lichtschutzprodukte UV-B-Filtersubstanzen als wesentliche Bestandteile, um insbesondere vor Sonnenbrand zu schützen. Zum Schutz gegen UV-B-Strahlung sind zahlreiche Verbindungen bekannt, wobei deren Absorptionsmaximum möglichst um 308 nm liegen sollte, da hier die höchste Erythemwirksamkeit der Sonnenstrahlung vorliegt. Typische UV-B-Filter sind z. B. Derivate des 3-Benzylidencamphers, der 4-Aminobenzoesäure, der Zimtsäure, der Salicylsäure, des Benzophenons sowie auch des 2-Phenylbenzimidazols.

Wie bereits dargestellt wird auch die Hautbräunung insbesondere durch UV-B-Licht angeregt. Hierbei wird von den Melanozyten in der Haut verstärkt Melanin gebildet, das letztlich die braune Färbung ausmacht. Diese sogenannte verzögerte Bräunung ("Delayed Tanning" oder "DT"), die im allgemeinen die erwünschte Bräunung darstellt, ist relativ lang anhaltend und stellt darüber hinaus auch einen hauteigenen UV-Schutz dar.

Lichtschutzprodukte mit einem Gehalt an UV-B-Filtersubstanzen schützen also nicht nur vor Sonnenbrand, sondern verlangsamen darüber hinaus - insbesondere bei hohem Lichtschutzfaktor — auch die Entstehung der natürlichen Hautbräunung.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

Darüber hinaus findet man viele der Schädigungen, die man früher typischerweise dem UV-B-Licht zugeschrieben hat, auch nach einer Bestrahlung mit UV-A-Licht. So sind z. B. Einschränkungen der zellulären Vitalität (welche man auch als "Cellperformance" bzw. Homöostase der Zellphysiologie etc. bezeichnen kann) auf UV-Licht im allgemeinen, aber insbesondere auf UV-A-Licht zurückzuführen. Unter anderem wird ferner auch die subzelluläre Organisation des Zytoskeletts durch UV-Licht stark beeinträchtigt und somit die "zelluläre Festigkeit" ("Cellular Firming") der Haut vermindert. Ferner kommt es auf zellulärer Ebene durch die Einwirkung von UV-Strahlung zu einer Vielzahl von Schädigungen, die durch Regeneration und Reparatur (Repair) sowohl auf Protein- als auch auf DNA-Ebene behoben werden müssen.

Ferner kommt es infolge eines Sonnenbades bzw. als Folge von Sonnenbestrahlung auch zu einer Austrocknung der Haut. Diese Austrocknung kann oberflächlicher Natur sein und überwiegend die Hornschicht betreffen. Sie kann sich aber auch als eine Art "Tiefenaustrocknung" auf die lebenden Zellschichten der Haut auswirken. Das Ausmaß der Oberflächenaustrocknung kann beispielsweise mit Hilfe der Corneometrie bestimmt werden. Das Ausmaß der Tiefenaustrockung (die Verminderung der Tiefenfeuchtigkeit) hingegen läßt sich z. B. durch Zellgrößen- bzw. Zellvolumenbestimmungen von lebenden Zellen den tieferen Hautschichten, insbesondere den lebenden Zellen der Epidermis, ermitteln.

Die neueren Erkenntnisse über die Wirkung der UV-A-Strahlen auf die Haut haben dazu geführt, daß man den Schutzmaßnahmen für diesen Strahlenbereich nun erhöhte Aufmerksamkeit widmet. Praktisch kein Sonnenschutzprodukt kommt mehr ohne wirksame UV-A-Filterwirkung aus, reine UV-B-Filterpräparate sind selten.

Auch der UV-A-Anteil des UV-Lichts leistet einen Beitrag zur Hautbräunung: Sofort nach Bestrahlung mit UV-A-Licht triit die sogenannte unmittelbare Bräunung (Immediate Pigment Darkening, IPD) auf, die allerdings auch schnell wieder verschwindet, keinen natürlichen UV-Schutz bietet und zudem eine eher unerwünschte gräuliche Bräune darstellt. Ferner ruft UV-A-Strahlung auch eine länger anhaltende Bräune (Permanent Pigment Darkening, PPD) hervor, welche von ihren Eigenschaften zwischen der verzögerten Bräunung DT und und der unmittelbaren Bräunung

IPD anzusiedeln ist (mit deutlichen Tendenzen zu IPD).

Auch zum Schutz gegen UV-A-Strahlung sind einige Verbindungen bekannt, wie insbesondere Dibenzoylmethanderivate. Weitere bekannte UV-A-Filtersubstanzen sind bestimmte wasserlösliche, sulfonierte UV-Filtersubstanzen, wie z. B. Phenylen-1,4-bis-(2-benzimidazyl)-3,3'-5,5'-tetrasulfonsäure und ihre Salze.

Eine gesunde (natürliche) Bräunung wird vom Verbraucher als erstrebenswert angesehen. Wichtig ist hierbei nicht nur ein generell "irgendwie dunklerer" Teint, sondern vielmehr eine sportliche, vitale und frische (natürliche) Hautbräunung, so wie man sie durch wohldosiertes (sanftes) Sonnenbaden erhält, welches nicht zu einem Sonnenbrand führt. Die Hautbräunung, welche beispielsweise nach einen Sonnenbank-Besuch auftritt, wird hingegen als unnatürlich oder gräulich empfunden. Auch der mit Selbstbräunern als alleinigen Pigmentierungsträgern erreichbare Farbton entspricht kaum der natürlichen gesunden Hautfarbe.

Beim Auftragen eines Sonnenschutzmittels auf die Haut können die ultravioletten Strahlen durch zwei Effekte abgeschwächt werden: zum einen durch Reflexion und Streuung der Strahlen an der Oberfläche von pulverförmigen Feststoffen (physikalischer Lichtschutz) und zum anderen durch Absorption an chemischen Substanzen (chemischer Lichtschutz). Je nachdem, welcher Wellenlängenbereich absorbiert wird, unterscheidet man dabei zwischen UV-B-Filtern (Absorptionsbereich 280 bis 320 nm), UV-A-Filtern (Absorptionsbereich 320 bis 400 nm) und Breitbandfiltern (Absorptionsbereich 290 bis ca. 380 nm). Zur Gruppe der Breitbandfilter gehören beispielsweise unsymmetrisch substituierte s-Triazin-Verbindungen, wie z. B. das 2,4-Bis-{[4-(2-ethyl-hexyloxy)-2-hydroxy]-phenyl}-6-(4-methoxyphenyl)-1,3,5-triazin (INCI: Bis-Ethylhexyloxylphenol Methoxyphenyl Triazin), bestimmte Benzophenone, wie z. B. das 2-Hydroxy-4-methoxy-benzophenon (INCI: Benzophenone 3) oder das 2,2'-Methylen-bis-(6-(2H-benzotriazol-2-yl)-4-(1,1,3,3-tetramethylbutyl)-phenol) (INCI: Methylene Bis-Benzotriazolyl Tetramethylenebutylphenol).

Die Einsatzkonzentration bekannter Lichtschutzfiltersubstanzen ist derzeit üblicherweise durch die Zusammensetzung der kosmetischen oder dermatologischen Lichtschutzzubereitung bedingt. Ziel der Entwicklung von Lichtschutzprodukten des Standes der Technik ist es dementsprechend, höhere Lichtschutzfaktoren bzw. UV-A-Schutzleistung zu erzielen und auf einfache und preiswerte Weise zu Zubereitungen zu gelangen, welche bei möglichst niedrigen Konzentrationen an herkömmlichen UV-Filtersubstanzen dennoch eine akzeptable oder sogar hohe UV-A und/oder UV-B-Schutzleistung erreichen. Der nach Anwendung und Bestrahlung erreichbare Farbton der Haut spielt hingegen bei der Entwicklung eines Sonnenschutzproduktes bislang keine Rolle.

Aufgabe der vorliegenden Erfindung war es daher, auf einfache Weise zu Lichtschutzzubereitungen zu gelangen, welche sich dadurch auszeichnen, daß der mit ihrer Hilfe erzielte Farbton möglichst nah am natürlichen Hautfarbton ist, wobei gleichzeitig die Vitalität sowie die Feuchtigkeit der Haut geschützt und gesteigert werden soll. Weitere Aufgabe der Erfindung war es, Zubereitungen zu finden, die den Hauteigenschutz vor UV-Strahlung steigern.

Überraschend und für den Fachmann nicht vorauszusehen war, daß kosmetische oder dermatologische Zubereitungen, dadurch gekennzeichnet, daß sie eine UVA-Balance von mehr als 15 aufweisen den Nachteilen des Standes der Technik abhelfen würde.

Besonders bevorzugt im Sinne der vorliegenden Erfindung sind Lichtschutzzubereitungen, die eine UVA-Balance von mehr als 15 aufweisen.

Die Zubereitungen gemäß der vorliegenden Erfindung stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die Zubereitungen gemäß der vorliegenden Erfindung zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität, eine überaus hohe UV-A- und UV-B-Schutzleistung sowie durch eine ausgezeichnete Wasser-, Schweiß-, Sand- und Abriebfestigkeit aus.

Die Zubereitungen im Sinne der vorliegenden Erfindung steigern und/oder verbessern ferner überraschend die zelluläre Vitalität der Hautzellen (welche man auch als "Cellperformance" bzw. Homöostase der Zellphysiologie etc. bezeichnen kann) und die "zelluläre Festigkeit" ("Cellular Firming") der Haut. Ferner verbessern sie auch das hauteigene (endogene) UV-Schutzsystem, insbesondere das endogene UV-A-Schutzsystem, und schützen ferner das Immunsystem der Haut. Auf diese Weise ermöglicht die Verwendung erfindungsgemäßer Zubereitungen einerseits einen unmittelbaren (akuten) Schutz in direktem zeitlichen Zusammenhang zu ihrer Anwendung und darüber hinaus einen sich über längere Sonnenexposition und Anwendung der Zubereitungen aufbauenden (endogenen) Schutz, der sich beispielsweise während der ersten Tage eines "Sonnenurlaubs" bildet und während dessen Verlauf immer besser wird. Durch die Verwendung von Zubereitungen im Sinne der vorliegenden Erfindung wird ferner das Risiko UV- bzw. lichtinduzierter Allergien (wie z. B. Sonnenallergie, polymorphe Lichtdermatose etc.) herabgesetzt, so daß sich die Zubereitungen hervorragend zur Anwendung bei lichtempfindlicher Haut eignen.

Durch die Verwendung erfindungsgemäßer Zubereitungen wird die ferner Hautfeuchtigkeit und die Tiefenfeuchtigkeit der Haut gesteigert, wobei diese hautbefeuchtende Pflege-Wirkung über mehrere Stunden bzw. sogar über den ganzen Tag oder noch länger anhält.

Ferner schützen die Zubereitungen gemäß der vorliegenden Erfindung Hautzellen und Haut vor vorzeitiger

Alterung und optisch wahrnehmbaren Veränderungen und unterstützen die Haut bei ihrer Regeneration und Reparatur (Repair) von Schädigungen, die durch die Einwirkung von UV-Strahlung auftreten können.

Es war ferner überraschend, daß die Zubereitungen im Sinne der vorliegenden Erfindung die Haut bereits unmittelbar nach bzw. bei ihrer Applikation wirksam vor den schädlichen Folgen einer UV-Bestrahlung schützen, wohingegen die Zubereitungen des Standes der Technik üblicherweise deutlich vor einem Sonnenbad aufgetragen werden sollten, um einen optimalen Schutz entfalten zu können.

Die UVA-Balance ist definiert als:

$$UVA\text{—}\ Balance = \frac{PPD_{in\ vitro} - 1}{SPF_{in\ vivo} - 1} \cdot 100$$

Der PPD-Wert wird üblicherweise *in vitro* bestimmt, beispielsweise wie bei Wendel *et al.* beschrieben (Wendel V. *et al.,* 2003, The influence of pre-irradiation on the predictability of *in vivo* UVA protection with a new *in vitro* method, *Photodermatol Photoimmunol Photomed,* 19:93-97). Hilfsweise kann der PPD-Wert alternativ auch *in vivo* bestimmt werden. Der Wert für den SPF (Sun Protection Factor, auch "LSF" für Lichtschutzfaktor genannt) wird gemäß Colipa *in vivo* bestimmt.

Besonders vorteilhafte Zubereitungen im Sinne der vorliegenden Erfindung weisen eine UVA-Balance von mehr als 22, insbesondere mehr als 30, ganz besonders bevorzugt mehr als 40 auf.

Es war ferner überraschend, daß die Verwendung von erfindungsgemäßen Zubereitungen sowohl ein gezieltes Bräunungsverhalten im Sinne einer natürlichen Bräunung als auch die Erhöhung des Hauteigenschutzes (d. h. das typische durch sonnensimuliertes UV-Licht / Sonnenlicht herbeigeführte Hautabsorptionsverhalten) ermöglicht.

Es war insbesondere überraschend, daß auch Zubereitungen mit demselben Lichtschutzfaktor (LSF) je nach Höhe der UV-A-Balance unterschiedliche Effekte zeigen, obwohl (bei identischem LSF) der UV-B-Anteil, der die Haut erreicht, selbstverständlich vergleichbar ist. Demnach wäre zu erwarten gewesen, daß auch die durch UV-B-Strahlung induzierten Effekte in der Haut (hinsichtlich natürlicher Bräunung und endogenem Schutz) vergleichbar sind. Stattdessen zeigt sich aber überraschenderweise, daß mit steigender UVA-Balance erstens die Bräunung natürlicher wird (und zwar: je höher die UVA-Balance, umso natürlicher der bei der Bräunung erzielte Farbton) und zweitens der endogene UV-Schutz, der sich in der Haut aufbaut, erhöht wird (je höher die UVA-Balance, umso höher die endogene Absorption im UV-Bereich, d. h. in einem Wellenlängenbereich zwischen 300 und 390nm).

"Unter natürlicher Farbton" bzw. "Natürlichkeit des Hautfarbtons" ist im Sinne der vorliegenden Erfindung zu verstehen, daß die mit Hilfe der erfindungsgemäßen Lichtschutzzubereitungen durch anschließende Bestrahlung mit UV- bzw. Sonnenlicht erhaltene Bräune mit der Hautbräune bzw. dem Hautfarbton vergleichbar ist, die/der durch eine sanfte Sonnenbräunung erreicht wird. Ob ein Hautfarbton als natürlich zu bezeichnen ist, läßt sich beispielsweise durch Ermittlung des Natural Tanning Factors (NTF) bestimmen.

Zur Beschreibung einer natürlichen Bräune und der Bestimmung des Natural Tanning Factors (NTF) werden mit einem handelsüblichen Spektrometer Remissionsspektren der Haut am hellen Innenarm und am sonnengebräunten Außenarm mittels Lichtleiter aufgenommen.

Das Spektrum des Außenarmes wird wellenlängenweise durch das Spektrum des Innenarmes dividiert, also normiert. Für die weitere Auswertung wird dann jeweils das normierte Spektrum herangezogen.

Vergleicht man nun die normierten Spektren für die unbehandelte, natürlich und sanft (d. h. unter Vermeidung von Sonnenbrand und dergleichen) gebräunte Haut mit denen, die nach mehrfacher Anwendung der erfindungsgemäßen Lichtschutzzubereitung und jeweils nachfolgender UV-Licht- bzw. Sonnenlichtexposition aufgenommen wurden, so kann man die Bräune als umso natürlicher bezeichnen, je mehr sich Form und Verlauf der Kurven gleichen.

Der NTF entspricht dem Verhältnis der Differenz (als Maß für die Steigung der Spektren in diesen Bereichen) der Reflektionswerte bei 410 nm ($R_{410}$) und bei 500 nm ($R_{500}$) zu der Differenz der Reflektionswerte bei 620 nm ($R_{620}$) und 750 nm ($R_{750}$):

$$NTF = (R_{410} — R_{500}) / (R_{620} — R_{750})$$

Ob mit Hilfe einer Lichtschutzzubereitung eine natürliche Bräune erzeugt wird, lässt sich also über einen statistischen Test ermitteln, in dem die individuellen NTFs sanft sonnengebräunter Haut mit denen der nach Auftrag der Lichtschutzzubereitung gebräunten Haut desselben oder eines anderen Probandenkollektivs verglichen werden. Gibt es zwischen den beiden Datensätzen keinen statistisch relevanten Unterschied, sind die Bräunungen als gleich zu bezeichnen, und dementsprechend kann die künstliche Bräune als natürlich wirkend angesehen werden.

Besonders vorteilhaft im Sinne der vorliegenden Erfindung ist es, wenn der NTF (im Sinne einer natürlichen Bräunung) zwischen 1 und ca. 3, bevorzugt zwischen 1,5 und ca. 3, besonders bevorzugt zwischen 2 und ca. 3 und

extrem bevorzugt ca. 3 ist.

Es ergibt sich von selbst, daß die Auswertung eines solchen Test auch rein visuell - beispielsweise durch den Probanden selbst - oder mit Hilfe eines anderen Meßverfahrens, mit dem sich Farbeindrücke ermitteln lassen, erfolgen kann.

Neben den dargestellten sichtbaren Effekten ("natürlicher Farbton" bzw. "Natürlichkeit des Hautfarbtons"), die - wie zuvor gezeigt - mit Hilfe des NTF beschrieben werden können, gibt es darüber hinaus noch den endogenen Hauteigenschutz vor UV-Licht, der durch die Verwendung der erfindungsgemäßen Formulierungen bei nachfolgender UV-Licht- oder Sonnenlichtexposition herbeigeführt wird.

"Unter natürlichem endogenem Hauteigenschutz" vor UV-Licht ist im Sinne der vorliegenden Erfindung zu verstehen, daß der mit Hilfe der Lichtschutzzubereitungen bei nachfolgender UV-Licht- bzw. Sonnenlichtexposition erhaltene endogene Hauteigenschutz mit dem endogenen bzw. natürlichen Hauteigenschutz vergleichbar ist, der durch eine sanfte Sonnenbräunung erreicht wird. Die Leistung des endogenen Hauteigenschutzes läßt sich beispielsweise durch Ermittlung des Endogenen Protection Factors (EPF) bestimmen.

Auch der EPF läßt sich aus den bereits beschriebenen Remissionsspektren ermitteln.

Vergleicht man nämlich die normierten Spektren für die unbehandelte, natürlich und sanft (d. h. unter Vermeidung von Sonnenbrand und dergleichen) gebräunte Haut mit denen, die nach mehrfacher Anwendung der erfindungsgemäßen Lichtschutzzubereitung und jeweils nachfolgender UV-Licht- bzw. Sonnenlichtexposition aufgenommen wurden, so kann man den natürlichen endogenen Schutz als umso besser bezeichnen, je mehr sich Form und Verlauf der Kurven im Wellenlängenbereich 290 bis 400 nm gleichen. Diesen Effekt kann man von der Bräunungstiefe unabhängiger machen, wenn man die zuletzt genannten Spektren bei auf die Intensität bei 700 nm normiert.

Zur rechnerischen Ermittlung des EPFs wird im Bereich von 310 bis 400 nm sowohl für die unbestrahlte als auch für die gebräunte Haut das Integral der Reflektion über die Wellenlänge gebildet. Der EPF entspricht der Differenz der so erhaltenen Werte für die unbestrahlten Hautareale und die Hautareale, die mehrfach mit den erfindungsgemäßen Lichtschutzformulierungen behandelt und jeweils anschließend mit UV- oder Sonnenlicht bestrahlt wurden:

$$\mathrm{EPF} = \int_{310}^{400} R\,d\lambda\ \mathrm{unbestrahlt} - \int_{310}^{400} R\,d\lambda\ \mathrm{bestrahlt}$$

Die statistische Bewertung des EPF erfolgt analog zum NTF.

Die erfindungsgemäße UVA-Balance läßt sich durch Kombination üblicher UV-Filtersubstanzen erreichen. Die UV-Filtersubstanzen werden vorteilhaft aus einer oder mehreren der folgenden Gruppen gewählt: UV-A-, UV-B- und/oder Breitbandfiltersubstanzen sowie organische und/oder anorganische Pigmente als UV-Filtersubstanzen.

Vorteilhafte UV-Filtersubstanzen im Sinne der vorliegenden Erfindung sind:

- Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

- Benzoxazol-Derivate, wie z. B. das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1 , 3,5-triazin (INCI: Ethylhexyl Triazone), welches von der BASF Aktiengesellschaft unter der Warenbezeichnung UVI-NUL® T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)ester;
- • Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter
- Ethylhexyl-2-cyano-3,3-diphenylacrylat (Octocrylen), welches von BASF unter der Bezeichnung Uvinul® N 539 T erhältlich ist.

Zur Erreichung eines hohen UV-A-Schutzes und eines sogenannten "kastenförmigen" Absorptionsspektrums" kann der Einsatz bestimmter UVA- und/oder Breitbandfiltersubstanzen wie z. B. dem 1,4-di (2-oxo-10-Sulfo-3-bornylidenmethyl)-Benzol und seinen Salzen (z. B. Terephthalylidene Dicamphor Sulfonic Acid (INCI)) und/oder Drometrizole Trisiloxane (INCI) sinnvoll sein. Erstaunlich und für den Fachmann nicht vorhersehbar, war jedoch, daß besonders vorteilhaft im Sinne der vorliegenden Erfindung Formulierungen sind, in denen die erwähnten UV-Filtersubstanzen Terephthalylidene Dicamphor Sulfonic Acid und Drometrizole Trisiloxane nicht enthalten sind.

Insbesondere ist es vorteilhaft, anstelle der UV-Filtersubstanzen Terephthalylidene Dicamphor Sulfonic Acid und Dromtrizole Trisiloxane organische UVA-Filtersubstanzen in Mengen einzusetzen, die dem Lichtschutzfaktor der Formulierungen angepaßt sind. Dies bedeutet, daß das Verhältnis der eingesetzten UV-A-Filtermengen zu den eingesetzten UV-B-Filtermengen ausreichend hoch sein muß. Bevorzugte Mischungsverhältnisse von UV-A-Filtern zu UV-B-Filtern liegen im Bereich von 0,1 bis 4 (bezogen auf die Massen der eingesetzen UV-Filter). Besonders bevorzugte Mischungsverhältnisse von UV-A-Filtern zu UV-B-Filtern liegen im Bereich von 0,2 bis 2,5 (bezogen auf die Massen der eingesetzen UV-Filter). Ganz besonders bevorzugte Mischungsverhältnisse von UV-A-Filtern zu UV-B-Filtern liegen im Bereich von 0,3 bis 1 (bezogen auf die Massen der eingesetzen UV-Filter). Zur Gruppe der UV-A-Filter gehören u. a. die oben beschriebenen Dibenzoylmethanderivate, die Phenylen-1,4-bis-(2-benzimidazyl)-3,3'-5,5'-tetrasulfonsäure und ihre Salze, das 1,4-di(2-oxo-10-Sulfo-3-bornylidenmethyl)-Benzol und dessen Salze, das 2,4-bis-[5-1(dimethylpropyl)benzoxazol-2-yl-(4-phenyl)-imino]-6-(2-ethylhexyl)-imino-1,3,5-triazin oder der 2-(4'-Diethylamino-2'-hydoxybenzoyl)-benzoesäurehexylester (auch: Aminobenzophenon). Zur Gruppe der UV-B-Filter gehören u. a. die oben beschriebenen Salze der 2-Phenylbenzimidazol-5-sulfonsäure, Dioctylbutylamidotriazon, Ethylhexyl Triazone, 3-Benzylidencampher-Derivate, Ester der Zimtsäure, Octocrylene oder an Polymere gebundene UV-B-Filter.

Weitere besonders vorteilhafte Zubereitungen im Sinne der vorliegenden Erfindung, die sich durch eine hohe bzw. sehr hohe UVA-Balance auszeichnen, enthalten bevorzugt sogenannte organische Breitbandfilter in Konzentrationen von 0,5 Gew.-% (bezogen auf das Gesamtgewicht der Zubereitung) und mehr. Zur Gruppe der Breitbandfilter gehören beispielsweise unsymmetrisch substituierte s-Triazin-Verbindungen, wie z. B. das 2,4-Bis-{[4-(2-ethyl-hexyloxy)-2-hydroxy]-phenyl}-6-(4-methoxyphenyl)-1,3,5-triazin, bestimmte Benzophenone, wie z. B. das 2-Hydroxy-4-methoxy-benzophenon oder das 2,2'-Methylen-bis-(6-(2H-benzotriazol-2-yl)-4-(1,1,3,3-tetramethylbutyl)-phenol). Weiterhin vorteilhaft ist der Einsatz von pigmentären, anorganischen UV-Filtern in Konzentrationen von 0,5 Gew.-% oder mehr. Zu dieser Gruppe gehören insbesondere Metalloxide wie Titandioxid und Zinkoxid, die sowohl Schutz vor UVB- als auch UVA-Strahlung bieten. Vorteilhaft im Sinne der vorliegenden Erfindung sind ebenfalls Formulierungen, die als UV-Filtersubstanzen ausschließlich Breitbandfilter und/oder pigmentäre, anorganische UV-Filter enthalten.

Besonders vorteilhafte Zubereitungen im Sinne der vorliegenden Erfindung enthalten zusätzlich zu einer oder mehreren UV-Filtersubstanzen ferner Kreatin/Kreatinin (z. B. in Verhältnissen 100 : 1, bevorzugt 50 : 1 bis 3 : 1, besonders bevorzugt im Verhältnis 3 : 1) und/oder Taurin.

Kosmetische oder dermatologische Zubereitungen im Sinne der vorliegenden Erfindung enthalten - bezogen auf das Gesamtgewicht der Zubereitungen - vorteilhaft 0,001 bis 10 Gew.-%, insbesondere 0,01 bis 5 Gew.-% an und ganz besonders 0,1 bis 2 Gew.-%, Kreatin/Kreatinin und/oder Taurin.

Die Haut wird unter dem Schutz der UV-Filter sowie durch die positiven Eigenschaften der angewandten Wirkstoffe Kreatin und/oder Taurin insbesondere hinsichtlich ihrer Vitalität positiv beeinflusst und insgesamt auch gegen die unerwünschten Effekte der vorzeitigen Hautalterung sowie den akut auftretenden Sonnenbrand hervorragend geschützt.

Gegenstand der Erfindung ist daher auch die

Verwendung von kosmetischen oder dermatologischen Lichtschutzzubereitungen mit einer UVA-Balance von mehr als 15 und einem Gehalt an Kreatin/Kreatinin und/oder Taurin zur Verbesserung und/oder Steigerung der Vitalität und des Eigenschutzes von Hautzellen sowie zum Schutz der Haut vor optisch wahrnehmbaren Veränderungen.

Die erfindungsgemäßen kosmetischen oder dermatologischen Zubereitungen können wie üblich zusammengesetzt sein und zur Behandlung und/oder Pflege der Haut und als Schminkprodukt in der dekorativen Kosmetik dienen. Die erfindungsgemäßen kosmetischen und/oder dermatologischen Zubereitungen können auch vorteilhaft in Form eines Produktes zur Pflege des Haars bzw. der Kopfhaut vorliegen, insbesondere eines Produktes zum Einlegen der Haare, das beim Fönen der Haare verwendet wird oder eines Frisier- und Behandlungsproduktes.

Zur Anwendung werden die erfindungsgemäßen Zubereitungen in der für Kosmetika üblichen Weise auf die Haut und/oder die Haare in ausreichender Menge aufgebracht.

Die Zubereitungen im Sinne der vorliegenden Erfindung können bevorzugt neben einer oder mehrerer Ölphasen zusätzlich eine oder mehrere Wasserphasen enthalten und beispielsweise in Form von O/W-, W/O/W- oder O/W/O-Emulsionen vorliegen. Solche Formulierungen können vorzugsweise auch Feststoff-Emulsionen (d. h. Emulsionen, welche durch Feststoffe stabilisiert sind, z. B. Pickering-Emulsionen), Gelemulsionen (Gelemulsionen oder Gelcremes sind sensorisch besonders leichte Produkte mit einem niedrigen Gehalt an Emulgatoren, Struktur- bzw. Gerüstbildnern (z. B. Fettalkoholen) und Lipiden), Hydrodispersionen oder auch Gele.

Vorteilhafte Zubereitungen im Sinne der vorliegenden Erfindung sind Emulsionen, insbesondere Mikropigment-Emulsionen sowie O/W- und W/O-Emulsionen. Ferner vorteilhaft im Sinne der vorliegenden Erfindung sind Sprayformulierungen.

## O/W-Emulsionen

O/W-Emulsionen im Sinne der vorliegenden Erfindung enthalten vorteilhaft einen oder mehrere Emulgatoren gewählt aus der Gruppe: Polyglyceryl-2 Dipolyhydroxystearat, Glycerylstearatcitrat, Glycerylstearat, Natriumcetearylsulfat, hydrogenated Coco-Glycerides sowie einen oder mehrere Coemulgatoren, wie beispielsweise Fettalkohole, insbesondere Cetearylalkohol und/oder Stearylakohol.

Die Ölphase erfindungsgemäßer O/W-Emulsionen enthält vorteilhaft Ölkomponenten gewählt aus der Gruppe: Butylenglykoldicaprylat/-dicaprat, Dicaprylylether, $C_{12-15}$-Alkylbenzoat, $C_{18-38}$-Fettsäuretriglycerid, Dibutyladipat, Cyclomethicon.

## W/O-Emulsionen

W/O-Emulsionen im Sinne der vorliegenden Erfindung enthalten vorteilhaft einen oder mehrere Emulgatoren gewählt aus der Gruppe: Polyglyceryl-2-dipolyhydroxystearat, PEG-30 Dipolyhydroxystearat.

Die Ölphase erfindungsgemäßer W/O-Emulsionen enthält vorteilhaft Ölkomponenten gewählt aus der Gruppe: Butylenglykoldicaprylat/-dicaprat, Paraffinum Liquidum, $C_{12-15}$-Alkylbenzoat, $C_{18-38}$-Fettsäuretriglycerid, Isopropylstearat, Cetyldimethicon.

## Sprühbare Emulsionen, insbesondere Mikroemulsionen

Auch sprühbare O/W-Emulsionen, insbesondere O/W-Mikroemulsionen sind besonders vorteilhaft im Sinne der vorliegenden Erfindung.

Die Tröpfchendurchmesser der gewöhnlichen "einfachen", also nichtmultiplen Emulsionen liegen im Bereich von ca 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 0,5 µm bis ca. 1 µm liegen, sind, wiederum ohne färbende Zusätze, bläulichweißgefärbt und opak. Solche "Makroemulsionen" haben für gewöhnlich ein hohe Viskosität.

Der Tröpfchendurchmesser von Mikroemulsionen im Sinne der vorliegenden Erfindung dagegen liegt im Bereich von etwa 50 bis etwa 500 nm. Derartige Mikroemulsionen sind bläulichweiß gefärbt bis transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von "Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Erfindungsgemäß vorteilhaft sind insbesondere O/W-Mikroemulsionen, welche mit Hilfe der sogenannten Phaseninversionstemperatur-Technologie erhältlich sind und mindestens einen Emulgator (Emulgator A) enthalten, welcher gewählt wird aus der Gruppe der Emulgatoren mit folgenden Eigenschaften:

- ihre Lipophilie ist abhängig von der Temperatur, dergestalt daß durch Erhöhung der Temperatur die Lipophilie zunimmt und durch Senkung der Temperatur die Lipophilie des Emulgators abnimmt.

Vorteilhafte Emulgatoren A sind z. B. polyethoxilierte Fettsäuren (PEG-100 Stearat, PEG-20 Stearat, PEG-150 Laurath, PEG-8 Distearat und dergleichen) und/oder polyethoxilierte Fettalkohole (Cetearath-12, Cetearath-20, Isoceteth-20, Beheneth-20, Laureth-9 etc.) und/oder Alkylpolyglycoside (Cetearyl Glycoside, Stearyl Glycoside, Palmityl Glycoside etc.).

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Die Tröpfchengrößen liegen in der Regel zwischen 20 und 500 nm.

Es ist im Sinn der vorliegenden Erfindung gegebenenfalls vorteilhaft, weitere, nicht unter die Definition des Emulgators A fallende, W/O und/oder O/W-Emulgatoren zu verwenden, beispielsweise um die Wasserfestigkeit der Zubereitungen gemäß der vorliegenden Erfindung zu erhöhen. Hier können z. B. Alkylmethiconcopolyole und/oder Alkyl-Dimethiconcopolyole (insbesondere Cetyl Dimethicone Copolyol, Lauryl Methicone Copolyol), W/O-Emulgatoren (wie z. B. Sorbitanstearat, Glycerylstearat, Glycerolstearat, Sorbitanoleat, Lecithin, Glycerylisostearat, Polyglyceryl-3-Oleat, Polyglyceryl-3 Diisostearat, PEG-7-hydriertes Ricinusöl, Polyglyceryl-4-isostearat, Acrylat/ $C_{10-30}$-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Glycol Distearat, Polyglyceryl-3-dipolyhydroxystearat) und/oder Fettsäureester der Schwefel- oder Phosphorsäure (Cetylphosphat, Trilaureth-4 Phosphat, Trioleth-8-Phosphat, Stearylphosphat, Cetearylsulfat etc.) verwendet werden.

Weitere vorteilhafte sprühbare O/W-Emulsionen im Sinne der vorliegenden Erfindung sind dünnflüssige kosmetische oder dermatologische Hydrodispersionen, welche mindestens eine Ölphase und mindestens eine Wasserphase enthalten, wobei die Zubereitung durch mindestens einen Gelbildner stabilisiert wird und nicht notwendigerweise Emulgatoren enthalten muß, aber einen oder mehrere Emulgatoren enthalten kann.

Vorteilhafte Gelbildner für derartige Zubereitungen sind beispielsweise Copolymere aus $C_{10-30}$-Alkylacrylaten und einem oder mehreren Monomeren der Acrylsäure, der Methacrylsäure oder deren Ester. Die INCI-Bezeichnung für solche Verbindungen ist Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 907, 910, 934, 940, 941, 951, 954, 980, 981, 1342, 1382, 2984 und 5984. ebenso die ETD-Typen 2020, 2050 und Carbopol Ultrez 10. Weitere vorteilhafte Gelbildner für derartige Zubereitungen sind Xanthan Gummi, Cellulose Derivate und Johannisbrotkernmehl.

Als mögliche (fakultative) Emulgatoren können ethoxilierte Fettalkohole oder ethoxilierte Fettsäuren (insbesondere PEG-100 Stearat, Ceteareth-20) und/oder andere nichtionische oberflächenaktive Substanzen verwendet werden.

Ferner vorteilhaft können die sehr dünnflüssigen bis sprühbaren Emulsionen auch W/O- bzw. Wasser-in-Silikonöl- (W/S-) Emulsionen sein. Insbesondere vorteilhaft sind W/O- bzw. W/S-Emulsionen, die

- mindestens einen Silikonemulgator (W/S) mit einem HLB-Wert ≤ 8 und/oder mindestens einen W/O-Emulgator mit einem HLB-Wert < 7 und
- mindestens einen O/W-Emulgator mit einem HLB-Wert > 10 enthalten.

Derartige Zubereitungen enthalten ferner mindestens 20 Gew.-% Lipide, wobei die Lipidphase vorteilhaft auch Silikonöle enthalten bzw. sogar ganz aus solchen bestehen kann.

Der oder die Silikonemulgatoren kann vorteilhaft aus der Gruppe der Alkylmethiconcopolyole und/oder Alkyl-Dimethiconcopolyole gewählt werden (z.B. Dimethiconcopolyole, welche von der Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden, Cetyl Dimethiconcopolyol [Goldschmidt AG / ABIL® EM 90], Cyclomethicon Dimethiconcopolyol [Goldschmidt AG / ABIL® EM 97], Laurylmethiconcopolyol [Dow Corning Ltd. / Dow Corning® 5200 Formulation Aid], Octyl Dimethicon Ethoxy Glucosid [Firma Wacker].

Der oder die W/O-Emulgatoren mit einem HLB-Wert < 7 kann vorteilhaft aus folgender Gruppe gewählt werden: Sorbitanstearat, Sorbitanoleat, Lecithin, Glyceryllanolat, Lanolin, hydriertem Ricinusöl, Glycerylisostearat, Polyglyceryl-3-Oleat, Pentaerythrithylisostearat, Methylglucosedioleat, Methylglucosedioleat im Gemisch mit Hydroxystearat und Bienenwachs, PEG-7-hydriertes Ricinusöl, Polyglyceryl-4-isostearat, Hexyllaurat, Acrylat/ $C_{10-30}$-Alkylacrylat-Crosspolymer, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat.

Der oder die O/W-Emulgatoren mit einem HLB-Wert > 10 kann vorteilhaft aus folgender Gruppe gewählt werden: Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat im Gemisch mit PEG-100 Stearat, Ceteareth-3, Isostearylglycerylether, Cetylstearylalkohol im Gemisch mit Natrium Cetylstearylsulfat, PEG-40-Stearat, Glycol Distearat, Polyglyceryl-2-PEG-4-Stearat, Ceteareth-12, Ceteareth-20, Ceteareth-30, Methyl-glucosesesquistearat, Steareth-10, PEG-20-Stearat, Steareth-21, Steareth-20, Isosteareth-20, PEG-45/ Dodecylglycol-Copolymer, Glycerylstearat SE, Ceteth-20, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquiisostearat, Ceteth-10, Isoceteth-20, Glycerylstearat im Gemisch mit Ceteareth-20, Ceteareth-12, Cetylstearylalkohol und Cetylpalmitat, PEG-30-Stearat, PEG-40-Stearat, PEG-100-Stearat.

Ferner vorteilhaft sind wäßrig-alkoholische Lösungen. Sie können von 0 Gew.-% bis 90 Gew.-% Ethanol enthalten. Wäßrig-alkoholische Lösungen können im Sinne der vorliegenden Erfindung vorteilhaft auch Lösungsvermittler enthalten, wie z. B. PEG-40 oder PEG-60 hydrogeniertes Ricinusöl.

Die Zubereitungen gemäß der vorliegenden Erfindung können vorteilhaft auch als kosmetische oder dermatologische Tränkungslösungen, mit welchen insbesondere wasserunlösliche Substrate - wie z. B. gewebte oder nichtgewebte Tücher - befeuchtet sind, verwendet werden. Derartige Tränkungslösungen sind vorzugsweise dünnflüssig, insbesondere sprühbar (wie z. B. PIT-Emulsionen, Hydrodispersionen, W/O-Emulsionen, Öle, wäßrige Lösungen etc.) und haben vorzugsweise eine Viskosität von weniger als 2000 mPa·s, insbesondere weniger als 1.500 mPa·s (Meßgerät: Haake Viskotester VT-02 bei 25 °C). Mit ihrer Hilfe sind beispielsweise kosmetische Sonnenschutztücher, Pflegetücher und dergleichen erhältlich, welche die Kombination eines weichen, wasserunlöslichen Materials mit der dünnflüssigen kosmetischen und dermatologischen Tränkungslösung darstellen.

## Schäume

Besonders vorteilhaft im Sinne der vorliegenden Erfindung sind ferner selbstschäumende, schaumförmige, nachschäumende oder schäumbare kosmetische und dermatologische Zubereitungen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische oder dermatologische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume — insbesondere dann, wenn die Gasbläschen zu klein sind, um unter einem Lichtmikroskop erkannt zu werden - auch an der starken Volumenzunahme des Systems erkennbar.

Derartige Zubereitungen enthalten im Sinne der vorliegenden Erfindung vorteilhaft ein Emulgatorsystem, welches aus

A. mindestens einem Emulgator A, gewählt aus der Gruppe der ganz-, teil- oder nicht neutralisierten, verzweigten

und/oder unverzweigten, gesättigten und/oder ungesättigten Fettsäuren mit einer Kettenlänge von 10 bis 40 Kohlenstoffatomen,

B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und

C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol ($C_{22}H_{45}OH$), Cetearylalkohol [eine Mischung aus Cetylalkohol ($C_{16}H_{33}OH$) und Stearylalkohol ($C_{18}H_{37}OH$)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

## Pickering- / feststoffstabilisierte Emulsionen

Besonders vorteilhaft im Sinne der vorliegenden Erfindung sind ferner kosmetische oder dermatologische Zubereitungen, welche nur durch feinstverteilte Feststoffteilchen stabilisiert sind. Solche "emulgatorfreien" Emulsionen werden auch als Pickering-Emulsionen bezeichnet.

In Pickering-Emulsionen kommt es zu einer Anreicherung des festen Stoffes an der Phasengrenze Öl/Wasser in Form einer Schicht, wodurch ein Zusammenfließen der dispersen Phasen verhindert wird. Von wesentlicher Bedeutung sind dabei insbesondere die Oberflächeneigenschaften der Feststoffpartikel, welche sowohl hydrophile als auch lipophile Eigenschaften zeigen sollten.

Vorteilhaft können die stabilisierenden Feststoffteilchen auch oberflächlich wasserabweisend behandelt ("gecoatet") sein, wobei ein amphiphiler Charakter dieser Feststoffteilchen gebildet werden bzw. erhalten bleiben soll. Die Oberflächenbehandlung kann darin bestehen, daß die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffteilchen wird vorzugsweise kleiner als 100 μm, besonders vorteilhaft kleiner als 50 μm gewählt. Dabei ist es im wesentlichen unerheblich, in welcher Form (Plättchen, Stäbchen, Kügelchen etc.) bzw. Modifikation die verwendeten Feststoffteilchen vorliegen.

Vorzugsweise werden die mikrofeinen Feststoffteilchen aus der Gruppe der amphiphilen Metalloxidpigmente gewählt. Vorteilhaft sind insbesondere:

- Titandioxide (gecoatet und ungecoatet): z. B. Eusolex T-2000 von der Fa. Merck, Titandioxid MT-100 Z von der Fa. Tayca Corporation
- Zinkoxide z. B. Z-Cote und Z-Cote HP1 von der BASF AG, MZ -300, MZ -500 und MZ-505M von der Fa. Tayca Corporation
- Eisenoxide

Des weiteren ist es vorteilhaft, wenn die mikrofeinen Feststoffteilchen aus der folgenden Gruppe gewählt werden: Bornitride, Stärkederivate (Tapioca Starch, Sodium Corn Starch Octynylsuccinat etc.), Talkum, Latexpartikel.

Es ist erfindungsgemäß vorteilhaft, wenn die feststoffstabilisierten Emulsionen deutlich weniger als 0,5 Gew.-% eines oder mehrerer Emulgatoren enthalten bzw. sogar gänzlich emulgatorfrei sind.

Die kosmetischen und dermatologischen Zubereitungen gemäß der Erfindung können kosmetische Hilfsstoffe

enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide und/oder β-Alanin sowie 8-Hexadecen-1,16-dicarbonsäure (Dioic acid, CAS-Nummer 20701-68-2; vorläufige INCI-Bezeichnung Octadecendioic acid).

Weitere vorteilhaft im Sinne der vorliegenden Erfindung einzusetzende Wirkstoffe sind solche, die den Zustand der Haut positiv beeinflussen, wie insbesondere Wirkstoffe zur positiven Beeinflussung der Altershaut, die die Entstehung von Falten oder auch bestehende Falten vermindern. Vorteilhaft sind insbesondere Biochinone, insbesondere Ubichinon Q10, Carnitin, Biotin, Isoflavon, Cardiolipin, Liponsäure, Anti Freezing Proteine, Hopfen- und Hopfen-Malz-Extrakte. Auch Mittel, die die Restrukturierung des Bindegewebes fördern, wie Isoflavonoide sowie Isoflavonoidhaltige Pflanzenextrakte - wie z. B. Soja- und Klee-Extrakte - können in den erfindungsgemäßen Formulierungen sehr gut verwendet werden. Auch zeigt sich, daß sich die Formulierungen in besonderer Weise eignen, Wirkstoffe zur Unterstützung der Hautfunktionen bei trockener Haut (wie beispielsweise Vitamin C, Biotin, Carnitin, Propionsäure, Grüntee-Extrakte, Eucalyptusöl, Harnstoff und Mineralsalze (wie z. B. NaCl, Meeresmineralien) sowie Osmolyte (wie z. B. Inositol, Betain, quartäre Ammoniumverbindungen)) zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süßholzes, Licochalcone, insbesondere Licochalcon A, Silymarin, Silyphos, Dexpanthenol, Inhibitoren des Prostaglandinstoffwechsels, insbesondere der Cyclooxygenase und des Leukotrienstoffwechsels, insbesondere der 5-Lipoxyaenase, aber auch des 5-Lipoxvgenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen. Beispielhaft sei erwähnt Tyrosinsulfat, Dioic acid (8-Hexadecen-1,16-dicarbonsäure) sowie Liponsäure und Liponamid, verschiedene Extrakte des Süßholzes, Koji - säure, Hydrochinon, Arbutin, Alpha-Arbutin, Deoxyarbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin, Pyridoxamin. Besonders bevorzugt sind ferner erfindungsgemäße Formulierungen, die weitere Wirkstoffe enthalten, die eine verstärkte oder schnellere Bräunung der Haut herbeiführen (Advanced Glycation Endproducts (AGE), Lipofuscine, NukleinsäureOligonukleotide, Purine und Pyrimidine, NO-freisetzende Substanzen), sei es mit oder ohne Einfluss von UV-Licht.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/ oder Isopropanol, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Pemulen TR-1 oder -2, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung. Die erfindungsgemäßen Formulierungen können ferner vorteilhaft neben Dihydroxyaceton auch Nußextrakte enthalten sowie weitere Substanzen, welche die Bräune erhalten oder erzeugen oder zusätzlich verstärken sollen.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch trans<u>e</u>pidermal <u>w</u>ater <u>l</u>oss (TEWL) genannt) zu reduzieren und/oder die Hydratation der Hornschicht positiv zu beeinflussen.

Vorteilhaft im Sinne der vorliegenden Erfindung werden der oder die Moisturizer gewählt aus der Gruppe: Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden. Es ist besonders vorteilhaft im Sinne der vorliegenden Erfindung, Glycerin als Moisturizer zu verwenden, bevorzugt in einer Konzentration von 0,05 bis 30 Gew.-%, besonders bevorzugt 1 bis 10 Gew.-%, jeweils bezogen auf das Gesamtgewicht der Zubereitung.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyl oleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE)* und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an $C_{12-15}$-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon *(Hallstar AB)* und/oder Diethylhexylnaphthalat *(Hallbrite* TQ oder Corapan TQ von Symrise).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

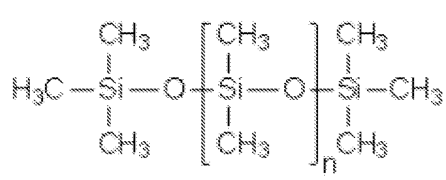

werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

**O/W-Emulsionen**

**Hoher Schutz (high protection), SPF 20**

| INCI | 1 | 2 | 3 | 4 | 5 | 6 | 7 | 8 | 9 |
|---|---|---|---|---|---|---|---|---|---|
| Alcohol Denat. | 3 | 4 | 2 | 0 | 3 | 5 | 5 | 5 | 5 |
| Acrylates/C10-30 Alkyl Acrylate Crosspolymer | 0,1 | 0,05 | 0,2 | 0,05 | 0 | 0 | 0 | 0 | 0 |
| Polyglyceryl-2 Dipolyhydroxystearate | 0,25 | 0,5 | 0,6 | 0,5 | 0 | 0 | 0 | 0 | 0 |
| Glyceryl Stearate Citrate | 1 | 2 | 3 | 2,5 | 0 | 0 | 0 | 0 | 0 |
| Glyceryl Stearate SE | 0 | 0 | 0 | 0 | 2 | 1 | 1 | 1 | 1 |
| Cetearyl Alcohol + PEG-40 Castor Oil + Sodium Cetearyl Sulfate | 0 | 0 | 0 | 0 | 5 | 2,5 | 2,5 | 2,5 | 2,5 |
| VP/Hexadecene Copolymer | 0,5 | 0,5 | 0,4 | 0,5 | 0,2 | 0,5 | 0,5 | 0,5 | 0,5 |
| Trisodium EDTA | 1 | 0,5 | 1 | 1 | 1 | 1 | 1 | 1 | 1 |
| Preservatives | q.s. | q.s. | q.s. | q.s. | q.s. | q.s. | q.s. | q.s. | q.s. |
| Terephthalidene Dicamphor Sulfonic Acid | | | | | | | 5 | 10 | 10 |
| Drometrizole Trisiloxane | | | | | | | 5 | 5 | 5 |
| Bis-Ethylhexyloxyphenol Methoxyphenyl Triazine | 0,5 | 1,4 | 1 | 2 | 0,5 | 1,4 | | | |
| Disodium Phenyl Dibenzimidazole Tetrasulfonate | | 1 | | 1,5 | | | | | |
| Ethylhexyl Triazone | 1 | 1,6 | 3,5 | 4 | 0 | 0 | 0 | 0 | 0 |
| Butyl Methoxydibenzoylmethane | 4,5 | 3,5 | 3,5 | 3 | 4,5 | 4,5 | 4,5 | 4,8 | 4,8 |
| Ethylhexyl Methoxycinnamate | 8 | 8 | 0 | 0 | 6 | 5 | | | |

(fortgesetzt)

| INCI | 1 | 2 | 3 | 4 | 5 | 6 | 7 | 8 | 9 |
|---|---|---|---|---|---|---|---|---|---|
| 2,4-bis-[5-1 (dimethylpropyl) benzoxazol-2-yl-(4-phenyl)-imino]-6-(2-ethylhexyl)-imino-1,3,5-triazin | | 1,5 | | | | | | 5 | 3 |
| Aminobenzophenon | 1 | | | | | | | 4 | 8 |
| Octocrylene | 0 | 0 | 0 | 0 | 2 | 3 | 10 | 10 | 10 |
| Phenylbenzimidazole Sulfonic Acid | 0 | 0 | 0 | 0 | 2 | 2 | | | |
| Ethylhexylglycerin | 0,5 | 0,5 | 0,5 | 0,5 | 0,5 | 0,5 | 0,5 | 0,5 | 0,5 |
| Glycerin | 5 | 7,5 | 7,5 | 6 | 2 | 8 | 8 | 8 | 8 |
| Hydrogenated Coco-Glycerides | 1 | 0 | 1 | 1 | 1 | 1 | 1 | 1 | 1 |
| Butylene Glycol Dicaprylate/Dicaprate | 3 | 2 | 9 | 8 | 4 | 2 | 2 | 2 | 2 |
| Dicaprylyl Ether | 2 | 3 | 1 | 4 | 1 | 2 | 2 | 2 | 2 |
| C12-15 Alkyl Benzoate | 5 | 8 | 7,5 | 6 | 8 | 4 | 4 | 4 | 4 |
| Dibutyl Adipate | 0 | 0 | 3 | 0 | 2 | 1 | 1 | 1 | 1 |
| Parfum | q.s. | q.s. | q.s. | q.s. | q.s. | q.s. | q.s. | q.s. | q.s. |
| Titanium Dioxide + Trimethoxycaprylylsilane | 3 | 3 | 3 | 3 | 3 | 3 | 5 | 5 | 5 |
| Cyclomethicone | 0 | 2 | 1 | 2 | 3 | 0 | 0 | 0 | 0 |
| Xanthan Gum | 0,2 | 0,1 | 0,4 | 0,2 | 0,4 | 0,25 | 0,25 | 0,25 | 0,25 |
| Stearyl Alcohol | 1 | 1,5 | 2 | 1,2 | 1 | 1,5 | 1,5 | 1,5 | 1,5 |
| Aqua | ad 100 | ad 100 | ad 100 | ad 100 | ad 100 | ad 100 | ad 100 | ad 100 | ad 100 |
| Creatinine | 0,05 | 0,1 | 0,03 | 0,05 | 0,06 | 0,05 | 0,05 | 0,05 | 0,05 |
| Dihydroxyacteon | | 3 | 5 | | | | | | |
| Tocopheryl Acetate | 0,5 | 0,2 | 0,5 | 0,5 | 0,5 | 0,5 | 0,5 | 0,5 | 0,5 |
| Creatine | 0,5 | 1 | 0,3 | 0,5 | 0,6 | 0,5 | 0,5 | 0,5 | 0,5 |

**O/W-Emulsionen**

**Sehr hoher Schutz (very high protection) SPF 30/40**

| INCI | 1 | 2 | 3 | 4 | 5 | 6 |
|---|---|---|---|---|---|---|
| Alcohol Denat. | 3 | 2 | 5 | 2 | 5 | 5 |
| Acrylates/C10-30 Alkyl Acrylate Crosspolymer | 0,02 | 0,05 | 0 | 0,05 | 0 | 0 |
| Polyglyceryl-2 Dipolyhydroxystearate | 0,4 | 0,5 | 0 | 0,5 | 0 | 0 |
| Glyceryl Stearate Citrate | 1,5 | 2,5 | 0 | 2,5 | 0 | 0 |

(fortgesetzt)

| INCI | 1 | 2 | 3 | 4 | 5 | 6 |
|---|---|---|---|---|---|---|
| Glyceryl Stearate SE | 0 | 0 | 1 | 0 | 1 | 1 |
| Cetearyl Alcohol + PEG-40 Castor Oil + Sodium Cetearyl Sulfate | 0 | 0 | 2,5 | 0 | 2,5 | 2,5 |
| VP/Hexadecene Copolymer | 0,5 | 0,5 | 0,5 | 0,5 | 0,5 | 0,5 |
| Trisodium EDTA | 1 | 1 | 1 | 1 | 1 | 1 |
| Preservatives | q.s. | q.s. | q.s. | q.s. | q.s. | q.s. |
| Terephthalidene Dicamphor Sulfonic Acid | | | | 8 | 10 | 10 |
| Drometrizole Trisiloxane | | | | 10 | 12 | 12 |
| Ethylhexyl Triazone | 1,5 | 4 | 0 | | | |
| Disodium Phenyl Dibenzimidazole Tetrasulfonate | 1 | | | | 1,2 | |
| Bis-Ethylhexyloxyphenol Methoxyphenyl Triazine | 2,5 | 2,5 | 2,5 | | | |
| Butyl Methoxydibenzoylmethane | 4 | 4,5 | 4 | 4,8 | 4,9 | |
| Ethylhexyl Methoxycinnamate | 9 | 0 | 6 | | | |
| Diethylhexyl Butamido Triazone | 0 | 1 | 0 | 1 | | |
| Phenylbenzimidazole Sulfonic Acid | 0 | 0 | 2 | | | |
| 2,4-bis-[5-1(dimethylpropyl)benzoxazol-2-yl-(4-phenyl) -imino]-6-(2-ethylhexyl)-imino-1,3,5-triazin | | | 1,5 | | | 9,2 |
| 2-(4'-Diethylamino-2'-hydoxybenzoyl)-benzoesäurehexylester | 1 | | | | | 8,6 |
| Octocrylene | 0 | 0 | 3 | 10 | 10 | 10 |
| Ethylhexylglycerin | 0,2 | 0,5 | 0,4 | 0,5 | 0,4 | 0,4 |
| Glycerin | 7,5 | 5 | 7,5 | 5 | 7,5 | 7,5 |
| Hydrogenated Coco-Glycerides | 1 | 1 | 1 | 1 | 1 | 1 |
| Dicaprylyl Ether | 1 | 4 | 2 | 4 | 2 | 2 |
| C12-15 Alkyl Benzoate | 2 | 5 | 4 | 5 | 4 | 4 |
| Butylene Glycol Dicaprylate/Dicaprate | 2,5 | 6,5 | 3 | 6,5 | 3 | 3 |
| Dibutyl Adipate | 0 | 0 | 2 | 0 | 2 | 2 |
| Parfum | q.s. | q.s. | q.s. | q.s. | q.s. | q.s. |
| Titanium Dioxide | 3 | 3 | 3 | 6 | 6 | 6 |
| Cyclomethicone | 0 | 2 | 0 | 2 | 0 | 0 |
| Xanthan Gum | 0,2 | 0,3 | 0,4 | 0,3 | 0,4 | 0,4 |
| Stearyl Alcohol | 1,5 | 1 | 1 | 1 | 1 | 1 |
| Aqua | ad 100 | ad 100 | ad 100 | ad 100 | ad 100 | ad 100 |
| Creatinine | 0,05 | 0,04 | 0,05 | 0,04 | 0,05 | 0,05 |
| Taurine | 0,9 | | | | | |
| Tocopheryl Acetate | 0,5 | 0,5 | 0,5 | 0,5 | 0,5 | 0,5 |
| Creatine | 0,5 | 0,4 | 0,5 | 0,4 | 0,5 | 0,5 |

**O/W-Emulsionen**

**Ultra hoher Schutz (ultra high protection), SPF 50+**

| INCI | 1 | 2 | 3 | 4 | 5 | 6 | 7 | 8 | 9 |
|---|---|---|---|---|---|---|---|---|---|
| Alcohol Denat. | 4 | 4 | 4 | 4 | 4 | 4 | 4 | 4 | 4 |
| Glyceryl Stearate SE | 1 | 1,5 | 2 | 2 | 1,2 | 2 | 1,2 | 1,2 | 1,2 |
| Cetearyl Alcohol + PEG-40 Castor Oil + Sodium Cetearyl Sulfate | 2,5 | 3,75 | 5 | 5 | 2,9 | 5 | 2,9 | 2,9 | 2,9 |
| VP/Hexadecene Copolymer | 0,5 | 0,5 | 0,8 | 0,7 | 0,2 | 0,7 | 0,2 | 0,2 | 0,2 |
| Trisodium EDTA | 1 | 1 | 1 | 1 | 1 | 1 | 1 | 1 | 1 |
| Preservatives | q.s. | q.s. | q.s. | q.s. | q.s. | q.s. | q.s. | q.s. | q.s. |
| 2,4-bis-[5-1 (dimethylpropyl) benzoxazol-2-yl-(4-phenyl) -imino]-6-(2-ethylhexyl)-imino-1,3,5-triazin | | 1,5 | | | | | | 9,4 | 8,2 |
| Aminobenzophenon | 1 | | | | | | | 6,7 | 8,9 |
| Octocrylene | | | | | | 10 | 10 | 10 | 10 |
| Terephthalidene Dicamphor Sulfonic Acid | | | | | | 8 | 9 | 9 | 9 |
| Drometrizole Trisiloxane | | | | | | 8 | 14 | 14 | 14 |
| Phenylbenzimidazole Sulfonic Acid | 2 | 2 | 2 | 2 | 2 | | | | |
| Disodium Phenyl Dibenzimidazole Tetrasulfonate | | 1,5 | | | | | | | |
| Diethylhexyl Butamido Triazone | 2 | 1 | 3 | 3 | 2 | | 5 | 5 | 5 |
| Bis-Ethylhexyloxyphenol Methoxyphenyl Triazine | 3 | 4 | 3 | 3 | 3 | | 3 | 3 | 3 |
| Ethylhexyl Triazone | 4 | 4 | 1,5 | 0,5 | 1,5 | | 2 | 2 | 2 |
| Butyl Methoxydibenzoylmethane | 4,0 | 3,0 | 4,5 | 4,5 | 4,5 | 4,9 | 4,5 | | 1 |
| Ethylhexyl Methoxycinnamate | 7,5 | 7,5 | 0 | 0 | 0 | | | | |
| Glycerin | 7,5 | 4 | 7,5 | 6 | 5 | 6 | 5 | 5 | 5 |
| Hydrogenated Coco-Glycerides | 1 | 0,5 | 1 | 0,5 | 0,6 | 0,5 | 0,6 | 0,6 | 0,6 |
| Dicaprylyl Carbonate | 1 | 2 | 3 | 4 | 2 | 4 | 2 | 2 | 2 |
| Butylene Glycol Dicaprylate/Dicaprate | 6,5 | 7,5 | 9 | 6 | 8 | 6 | 8 | 8 | 8 |
| C12-15 Alkyl Benzoate | 0 | 0 | 5 | 4 | 2 | 4 | 2 | 2 | 2 |

(fortgesetzt)

| INCI | 1 | 2 | 3 | 4 | 5 | 6 | 7 | 8 | 9 |
|---|---|---|---|---|---|---|---|---|---|
| Parfum | q.s. | q.s. | q.s. | q.s. | q.s. | q.s. | q.s. | q.s. | q.s. |
| Titanium Dioxide | 5 | 5 | 5 | 5 | 5 | 10 | 10 | 10 | 10 |
| Xanthan Gum | 0,2 | 0,4 | 0 | 0,3 | 0,1 | 0,3 | 0,1 | 0,1 | 0,1 |
| C18-36 Acid Triglyceride | 1 | 0,5 | 0 | 1 | 0,2 | 1 | 0,2 | 0,2 | 0,2 |
| Cetyl Alcohol | 1 | 1,5 | 1,2 | 0,5 | | 0,5 | | | |
| Aqua | ad 100 | ad 100 | ad 100 | ad 100 | ad 100 | ad 100 | ad 100 | ad 100 | ad 100 |
| Tocopheryl Acetate | 0,5 | 0,5 | 0,5 | 0,5 | 0,5 | 0,5 | 0,5 | 0,5 | 0,5 |

**Sonnensprays**

**Hoher Schutz (high protection), SPF 20**

| INCI | 1 | 2 | 3 | 4 | 5 | 6 | 7 | 8 |
|---|---|---|---|---|---|---|---|---|
| Alcohol Denat. | 2 | 4 | 3,5 | 5 | 3,5 | 5 | 3,5 | 5 |
| Ceteareth-20 | 1 | 1 | 1 | 1 | 1 | 1 | 1 | 1 |
| VP/Hexadecene Copolymer | 0,5 | 0,5 | 0,5 | 0,5 | 0,5 | 0,5 | 0,5 | 0,5 |
| Trisodium EDTA | 1 | 1 | 1 | 1 | 1 | 1 | 1 | 1 |
| Preservatives | q.s. | q.s. | q.s. | q.s. | q.s. | q.s. | q.s. | q.s. |
| Bis-Ethylhexyloxyphenol Methoxyphenyl Triazine | 0,5 | 1 | 1 | 1,5 | | 1,5 | | 1,5 |
| Disodium Phenyl Dibenzimidazole Tetrasulfonate | | | 1 | | | | 1 | |
| Phenylbenzimidazole Sulfonic Acid | 2 | 1,5 | 2 | 2 | | 2 | | 2 |
| Ethylhexyl Triazone | 3 | 0 | 3 | 0 | 3 | 0 | 3 | 0 |
| Butyl Methoxydibenzoylmethane | 3,8 | 3,5 | 4,0 | 4,5 | 4,5 | 4,8 | | |
| Ethylhexyl Methoxycinnamate | 0 | 9 | 0 | 0 | | 0 | | 0 |
| 2,4-bis-[5-1(dimethylpropyl) benzoxazol-2-yl-(4-phenyl)-imino]-6-(2-ethylhexyl)-imino-1,3,5-triazin | 1 | | | | | | 8,8 | 9 |
| 2-(4'-Diethylamino-2'-hydoxybenzoyl)-benzoesäurehexylester | | 2 | | | | | 5,9 | 6,8 |
| Octocrylene | 0 | 0 | 0 | 3 | 10 | 10 | 10 | 10 |
| Ethylhexyl Salicylate | 0 | 0 | 0 | 4 | | 4 | | 4 |
| Terephthalidene Dicamphor Sulfonic Acid | | | | | 6 | 8,5 | 6 | 8,5 |
| Drometrizole Trisiloxane | | | | | 10 | 12 | 10 | 12 |
| Titanium Dioxide | | | | | 4 | 5 | 4 | 5 |

(fortgesetzt)

| INCI | 1 | 2 | 3 | 4 | 5 | 6 | 7 | 8 |
|---|---|---|---|---|---|---|---|---|
| Glycerin | 7,5 | 5 | 8 | 6 | 8 | 6 | 8 | 6 |
| C12-15 Alkyl Benzoate | 4 | 0 | 5 | 0 | 5 | 0 | 5 | 0 |
| Butylene Glycol Dicaprylate/ Dicaprate | 8 | 4 | 7,5 | 3 | 7,5 | 3 | 7,5 | 3 |
| Parfum | q.s. | q.s. | q.s. | q.s. | q.s. | q.s. | q.s. | q.s. |
| Acrylates/C10-30 Alkyl Acrylate Crosspolymer | 0,2 | 0,4 | 0,3 | 0,5 | 0,3 | 0,5 | 0,3 | 0,5 |
| C18-36 Acid Triglyceride | 0,5 | 1 | 0,3 | 1 | 0,3 | 1 | 0,3 | 1 |
| Aqua | ad 100 | ad 100 | ad 100 | ad 100 | ad 100 | ad 100 | ad 100 | ad 100 |
| Creatinine | 0,06 | 0,05 | 0,05 | 0 | 0,05 | 0 | 0,05 | 0 |
| Dihydroxyacteon | | 4 | | | | | | |
| Tocopheryl Acetate | 0,5 | 0,5 | 0,5 | 0,5 | 0,5 | 0,5 | 0,5 | 0,5 |
| Creatine | 0,6 | 0,5 | 0,5 | 0 | 0,5 | 0 | 0,5 | 0 |

**Sonnensprays**

**Sehr hoher oder ultra hoher Schutz (very high or ultra high protection), SPF 30/40/50/50+**

| INCI | 1 | 2 | 3 | 4 | 5 | 6 | 7 | 8 |
|---|---|---|---|---|---|---|---|---|
| Alcohol Denat. | 2 | 3 | 4 | 5 | 4 | 5 | 4 | 5 |
| Ceteareth-20 | 0,5 | 1,5 | 1,2 | 1 | 1,2 | 1 | 1,2 | 1 |
| VP/Hexadecene Copolymer | 0,4 | 0,5 | 0,3 | 0,2 | 0,3 | 0,2 | 0,3 | 0,2 |
| Trisodium EDTA | 1 | 1 | 1 | 1 | 1 | 1 | 1 | 1 |
| Preservatives | q.s. | q.s. | q.s. | q.s. | q.s. | q.s. | q.s. | q.s. |
| Diethylhexyl Butamido Triazone | 2 | 0 | 2 | 2 | | | | |
| Disodium Phenyl Dibenzimidazole Tetrasulfonate | | 1 | | | | | 1,5 | |
| Phenylbenzimidazole Sulfonic Acid | 2 | 2 | 2 | 2 | | | | |
| Ethylhexyl Triazone | 3 | 3 | 0 | 0 | 5 | 0 | 5 | 0 |
| Bis-Ethylhexyloxyphenol Methoxyphenyl Triazine | 3 | 2,5 | 2 | 2 | | 2 | | 2 |
| Butyl Methoxydibenzoylmethane | 4,5 | 3,9 | 4,5 | 4,5 | 4,9 | 4,8 | 2 | |
| Ethylhexyl Methoxycinnamate | 0 | 6 | 3 | 3 | | 3 | | 3 |
| 2,4-bis-[5-1(dimethylpropyl) benzoxazol-2-yl-(4-phenyl) -imino]-6-(2-ethylhexyl)-imino-1,3,5-triazin | | 1 | | | | | 8,8 | 7,6 |
| Aminobenzophenon | 0,5 | | | | | | 9,6 | 8,4 |
| Octocrylene | 0 | 0 | 3 | 3 | 10 | 10 | 10 | 10 |

(fortgesetzt)

| INCI | 1 | 2 | 3 | 4 | 5 | 6 | 7 | 8 |
|---|---|---|---|---|---|---|---|---|
| Terephthalidene Dicamphor Sulfonic Acid | | | | | 7 | 9,8 | 7 | 9,8 |
| Drometrizole Trisiloxane | | | | | 8 | 11 | 8 | 11 |
| Glycerin | 7,5 | 5 | 6 | 6 | 6 | 6 | 6 | 6 |
| C12-15 Alkyl Benzoate | 3 | 0 | 0 | 0 | 0 | 0 | 0 | 0 |
| Butylene Glycol Dicaprylate/ Dicaprate | 8 | 7 | 9 | 6 | 9 | 6 | 9 | 6 |
| Parfum | q.s. | q.s. | q.s. | q.s. | q.s. | q.s. | q.s. | q.s. |
| Titanium Dioxide | 0 | 0 | 2 | 1 | 10 | 7 | 10 | 7 |
| Acrylates/C10-30 Alkyl Acrylate Crosspolymer | 0,1 | 0,2 | 0,4 | 0,4 | 0,4 | 0,4 | 0,4 | 0,4 |
| C18-36 Acid Triglyceride | 1 | 1 | 1 | 1 | 1 | 1 | 1 | 1 |
| Aqua | ad 100 | ad 100 | ad 100 | ad 100 | ad 100 | ad 100 | ad 100 | ad 100 |
| Creatinine | 0,05 | 0,05 | 0 | 0 | 0 | 0,05 | 0 | 0,05 |
| Tocopheryl Acetate | 0,5 | 0,5 | 0,5 | 0,5 | 0,5 | 0,5 | 0,5 | 0,5 |
| Taurine | | 1,1 | | 0,8 | | | | |
| Creatine | 0,2 | 0,5 | 0 | 0 | 0 | 0 | 0 | 0 |
| Panthenol | 0 | 0 | 1,4 | 1,4 | 1,4 | 1,4 | 1,4 | 1,4 |

**W/O-Emulsionen**

**Hoher Schutz (high protection), SPF 20**

| INCI | 1 | 2 | 3 | 4 | 5 | 6 |
|---|---|---|---|---|---|---|
| Alcohol Denat. | 1 | 2 | 1 | 2 | 1 | 2 |
| Polyglyceryl-2 Dipolyhydroxystearate | 4 | 5,5 | 4 | 5,5 | 4 | 5,5 |
| Trisodium EDTA | 1 | 1 | 1 | 1 | 1 | 1 |
| Preservatives | q.s. | q.s. | q.s. | q.s. | q.s. | q.s. |
| Paraffinum Liquidum | 0 | 1,8 | 0 | 1,8 | 0 | 1,8 |
| Ethylhexyl Triazone | | | 1 | | | |
| Diethylhexyl Butamido Triazone | 0,5 | 0 | | | | |
| Bis-Ethylhexyloxyphenol Methoxyphenyl Triazine | 1 | 1 | | | | |
| 2,4-bis-[5-1(dimethylpropyl)benzoxazol-2-yl-(4-phenyl) -imino]-6-(2-ethylhexyl)-imino-1,3,5-triazin | | 0,5 | | | 9 | 8 |
| Aminobenzophenon | | 0,5 | 0,8 | | 6,4 | 5,5 |
| Octocrylene | 2,5 | 6 | 10 | 10 | 10 | 10 |
| Terephthalidene Dicamphor Sulfonic Acid | | | 8 | | 8 | |
| Drometrizole Trisiloxane | | | 12 | | 12 | |

(fortgesetzt)

| INCI | 1 | 2 | 3 | 4 | 5 | 6 |
|---|---|---|---|---|---|---|
| Disodium Phenyl Dibenzimidazole Tetrasulfonate | | | 1 | | | |
| Butyl Methoxydibenzoylmethane | 3,5 | 0,9 | 4 | 4,7 | | |
| Ethylhexyl Methoxycinnamate | 7,5 | 0 | 7,5 | 0 | 7,5 | 0 |
| Ethylhexylglycerin | 0,5 | 0,5 | 0,5 | 0,5 | 0,5 | 0,5 |
| Butylene Glycol | 5 | 5 | 5 | 5 | 5 | 5 |
| Glycerin | 7,5 | 5 | 7,5 | 5 | 7,5 | 5 |
| Isopropyl Stearate | 3 | 6 | 3 | 6 | 3 | 6 |
| Butylene Glycol Dicaprylate/Dicaprate | 7,5 | 8 | 7,5 | 8 | 7,5 | 8 |
| C12-15 Alkyl Benzoate | 9,5 | 5 | 9,5 | 5 | 9,5 | 5 |
| Parfum | q.s. | q.s. | q.s. | q.s. | q.s. | q.s. |
| Sodium Starch Octenylsuccinate | 0,4 | 0,55 | 0,4 | 0,55 | 0,4 | 0,55 |
| Titanium Dioxide | 4,5 | 2,5 | 5 | 6 | 5 | 6 |
| Magnesium Sulfate | 0,3 | 0,2 | 0,3 | 0,2 | 0,3 | 0,2 |
| Cetyl Dimethicone | 0 | 0,5 | 0 | 0,5 | 0 | 0,5 |
| Glycine | 0,2 | 0,5 | 0,2 | 0,5 | 0,2 | 0,5 |
| C18-36 Acid Triglyceride | 0,5 | 1 | 0,5 | 1 | 0,5 | 1 |
| Aqua | ad 100 | ad 100 | ad 100 | ad 100 | ad 100 | ad 100 |
| Creatine | | 0,5 | | | | |
| Creatinine | | 0,05 | | | | |
| Taurine | 1 | | | | | |
| Tocopheryl Acetate | 0,5 | 0,5 | 0,5 | 0,5 | 0,5 | 0,5 |
| Panthenol | 0 | 0,75 | 0 | 0,75 | 0 | 0,75 |

**W/O-Emulsionen**

**Sehr hoher oder ultra hoher Schutz (very high or ultra high protection), SPF 30/40/50/50+**

| INCI | 1 | 2 | 3 | 4 | 5 | 6 | 7 | 8 |
|---|---|---|---|---|---|---|---|---|
| Alcohol Denat. | 1 | 2 | 3 | 2 | 3 | 2 | 3 | 2 |
| Polyglyceryl-2 Dipolyhydroxystearate | 4 | 5,8 | 5 | 0 | 5 | 0 | 5 | 0 |
| PEG-30 Dipolyhydroxystearate | 0 | 0 | 0 | 3,5 | 0 | 3,5 | 0 | 3,5 |
| Trisodium EDTA | 1 | 1 | 1 | 1 | 1 | 1 | 1 | 1 |
| Preservative system | q.s. | q.s. | q.s. | q.s. | q.s. | q.s. | q.s. | q.s. |
| Diethylhexyl Butamido Triazone | 0,5 | | 1 | | 0 | 2 | 0 | 2 |
| Ethylhexyl Triazone | | 0,5 | | 2 | | | | |
| Bis-Ethylhexyloxyphenol Methoxyphenyl Triazine | 1 | 2 | 3 | 2,5 | 3 | 2,5 | 3 | 2,5 |

(fortgesetzt)

| INCI | 1 | 2 | 3 | 4 | 5 | 6 | 7 | 8 |
|---|---|---|---|---|---|---|---|---|
| 2,4-bis-[5-1(dimethylpropyl) benzoxazol-2-yl-(4-phenyl) -imino]-6-(2-ethylhexyl)-imino-1,3,5-triazin | | 1 | | | | | 5,9 | 9,4 |
| 2-(4'-Diethylamino-2'-hydoxybenzoyl)-benzoesäurehexylester | | | 0,5 | | | | 9,5 | 9,3 |
| Octocrylene | 2,5 | 3,5 | 2,5 | 3 | 10 | 3 | 10 | 3 |
| Terephthalidene Dicamphor Sulfonic Acid | | | | | 9,5 | | 9,5 | |
| Drometrizole Trisiloxane | | | | | 12 | | 12 | |
| Disodium Phenyl Dibenzimidazole Tetrasulfonate | 0,5 | | | | | | | 1 |
| Butyl Methoxydibenzoylmethane | 3,5 | 4,5 | 0,9 | 4 | 4,7 | 4 | 2 | |
| Ethylhexyl Methoxycinnamate | 7,5 | 7,5 | 0,5 | 0,5 | 0,5 | 0,5 | 0,5 | 0,5 |
| Ethylhexylglycerin | 0,5 | 0,5 | 0,5 | 0,5 | 0,5 | 0,5 | 0,5 | 0,5 |
| Butylene Glycol | 2,5 | 2,5 | 5 | 4 | 5 | 4 | 5 | 4 |
| Glycerin | 7,5 | 7,5 | 5 | 6 | 5 | 6 | 5 | 6 |
| Isopropyl Stearate | 4 | 2,5 | 8 | 5 | 8 | 5 | 8 | 5 |
| Butylene Glycol Dicaprylate/ Dicaprate | 7 | 6,5 | 8 | 6 | 8 | 6 | 8 | 6 |
| C12-15 Alkyl Benzoate | 9 | 7 | 8 | 5 | 8 | 5 | 8 | 5 |
| Parfum | q.s. | q.s. | q.s. | q.s. | q.s. | q.s. | q.s. | q.s. |
| Titanium Dioxide | 4,5 | 5 | 4,5 | 6 | 10 | 6 | 10 | 6 |
| Magnesium Sulfate | 0,3 | 0,1 | 0,2 | 0,2 | 0,2 | 0,2 | 0,2 | 0,2 |
| Cetyl Dimethicone | 0 | 0,5 | 0 | 0,2 | 0 | 0,2 | 0 | 0,2 |
| Dihydroxyacteon | | | 5 | | | | | |
| Glycine | 0,3 | 0,4 | 0,5 | 0,5 | 0,5 | 0,5 | 0,5 | 0,5 |
| C18-36 Acid Triglyceride | 0,4 | 0,6 | 0,5 | 0,45 | 0,5 | 0,45 | 0,5 | 0,45 |
| Tocopheryl Acetate | 0,5 | 0,5 | 0,5 | 0,5 | 0,5 | 0,5 | 0,5 | 0,5 |
| Panthenol | 0 | 0 | 1,5 | 1,5 | 1,5 | 1,5 | 1,5 | 1,5 |
| Aqua | ad 100 | ad 100 | ad 100 | ad 100 | ad 100 | ad 100 | ad 100 | ad 100 |

**Lichtschutzemulsionen**

**Sehr hoher Schutz (very high protection) SPF 30/40/50**

| INCI | 1 | 2 | 3 | 4 | 5 | 6 |
|---|---|---|---|---|---|---|
| Alcohol Denat. | 3 | 3 | 0 | 3 | 0 | 0 |
| Glyceryl Stearate SE | 1,5 | 1 | 0 | 1 | 0 | 0 |

(fortgesetzt)

| INCI | 1 | 2 | 3 | 4 | 5 | 6 |
|---|---|---|---|---|---|---|
| PEG-30 Dipolyhydroxystearate | 1 | 0 | 0 | 0 | 0 | 0 |
| Cetearyl Alcohol + PEG-40 Castor Oil + Sodium Cetearyl Sulfate | 3,75 | 2,5 | 0 | 2,5 | 0 | 0 |
| Cetyl PEG/PPG-10/1 Dimethicone | 0 | 0,8 | 3,8 | 0,8 | 3,8 | 3,8 |
| Polyglyceryl-2 Dipolyhydroxystearate | 0 | 0 | 2 | 0 | 2 | 2 |
| Trisodium EDTA | 1 | 1 | 0 | 1 | 0 | 0 |
| Preservative system | q.s. | q.s. | q.s. | q.s. | q.s. | q.s. |
| Cera Microcristallina + Paraffinum Liquidum | 0 | 0 | 3 | 0 | 3 | 3 |
| 2,4-bis-[5-1(dimethylpropyl)benzoxazol-2-yl-(4-phenyl) -imino]-6-(2- ethylhexyl)-imino-1,3,5-triazin | | | | 8,9 | 8,1 | 8,9 |
| 2-(4'-Diethylamino-2'-hydoxybenzoyl)-benzoesäurehexylester | | | | 5,9 | 6,4 | 9 |
| Terephthalidene Dicamphor Sulfonic Acid | | | | | | 9,2 |
| Drometrizole Trisiloxane | | | | | | 13 |
| Glycerin | 7,5 | 3 | 5 | 3 | 5 | 5 |
| Butylene Glycol | 0 | 0 | 5 | 0 | 5 | 5 |
| Hydrogenated Coco-Glycerides | 0,5 | 0,5 | 0 | 0,5 | 0 | 0 |
| Dicaprylyl Carbonate | 1 | 2,35 | 0 | 2,35 | 0 | 0 |
| Caprylic/Capric Triglyceride | 5 | 4 | 7 | 4 | 7 | 7 |
| Butylene Glycol Dicaprylate/Dicaprate | 7,5 | 8 | 6 | 8 | 6 | 6 |
| C12-15 Alkyl Benzoate | 0 | 0 | 1,5 | 0 | 1,5 | 1,5 |
| Dicaprylyl Ether | 0 | 0 | 5 | 0 | 5 | 5 |
| Parfum | 0 | 0 | q.s. | 0 | q.s. | q.s. |
| Zinc Oxide + Dimethicone | 7 | 8 | 8 | 15 | 10 | 10 |
| Titanium Dioxide | 8 | 9 | 9 | 20 | 15 | 15 |
| Magnesium Sulfate | 0 | 0 | 0,3 | 0 | 0,3 | 0,3 |
| Cyclomethicone | 0 | 0 | 4 | 0 | 4 | 4 |
| Glycine | 0 | 0 | 0,5 | 0 | 0,5 | 0,5 |
| Xanthan Gum | 0,2 | 0,3 | 0 | 0,3 | 0 | 0 |
| C18-36 Acid Triglyceride | 0,5 | 1 | 0 | 1 | 0 | 0 |
| Cetyl Alcohol | 1 | 1,5 | 0 | 1,5 | 0 | 0 |
| Aqua | ad 100 | ad 100 | ad 100 | ad 100 | ad 100 | ad 100 |
| Tocopheryl Acetate | 0,5 | 0,5 | 0,5 | 0,5 | 0,5 | 0,5 |
| Panthenol | 0 | 0 | 1,4 | 0 | 1,4 | 1,4 |

## Patentansprüche

1. Kosmetische oder dermatologische Zubereitungen, **dadurch gekennzeichnet, daß** sie eine UVA-Balance von mehr als 15 aufweisen.

2. Kosmetische oder dermatologische Zubereitungen, **dadurch gekennzeichnet, daß** sie eine UVA-Balance von mehr als 22 aufweisen.

3. Kosmetische oder dermatologische Zubereitungen, **dadurch gekennzeichnet, daß** sie eine UVA-Balance von mehr als 30 aufweisen.

4. Kosmetische oder dermatologische Zubereitungen, **dadurch gekennzeichnet, daß** sie eine UVA-Balance von mehr als 40 aufweisen.

5. Zubereitung nach einem der vorhergehenden Ansprüche, **dadurch gekennzeichnet, daß** sie als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure enthält.

6. Zubereitung nach einem der vorhergehenden Ansprüche, **dadurch gekennzeichnet, daß** sie mindestens eine UV-Filtersubstanz, gewählt aus der folgenden Gruppe, enthält:

   Wasserlösliche UV-Filtersubstanzen, bei Raumtemperatur flüssige UV-Filter, organische und/oder anorganische Pigmente, Breitbandfilter (wie Bis-Resorcinyltriazinderivate, insbesondere das 2,4-Bis-{[4-(2-Ethyl-hexyloxy)-2-hydroxy]-phenyl}-6-(4-methoxyphenyl)-1,3,5-triazin) und/oder Benzotriazole.

7. Zubereitung nach einem der vorhergehenden Ansprüche, **dadurch gekennzeichnet, daß** sie mindestens eine UV-Filtersubstanz, gewählt aus der Benzoxazol-Derivate (insbesondere das 2,4-bis-[5-1(dimethylpropyl)benzoxazol-2-yl-(4-phenyl)-imino]-6-(2-ethylhexyl)-imino-1,3,5-triazin und/oder aus der Gruppe der Hydroxybenzophenone (insbesondere der 2-(4'-Diethylamino-2'-hydoxybenzoyl)-benzoesäurehexylester) enthält.

8. Zubereitung nach einem der vorhergehenden Ansprüche, **dadurch gekennzeichnet, dass** der Lichtschutzfaktor (ermittelt gemäß COLIPA-Methode) der Formulierungen mindestens 20 beträgt.

9. Zubereitung nach einem der Ansprüche 1 bis 8, **dadurch gekennzeichnet, daß** sie ferner Kreatin/Kreatinin und/oder Taurin enthalten.

10. Verwendung von Zubereitungen nach einem der Ansprüche 1 bis 9 zur Steigerung und/oder Verbesserung des natürlichen, hauteigenen (endogenen) UV-Schutzsystems, insbesondere des endogenen UV-A-Schutzes.

11. Verwendung von Zubereitungen nach einem der Ansprüche 1 bis 9 zur Steigerung und/oder Verbesserung des Schutzes der Haut und der Hautzellphysiologie, insbesondere im Sinne einer besser geschützten Vitalität, eines "cellular firmings" bzw. einer "Cellperformance".

12. Verwendung von Zubereitungen nach einem der Ansprüche 1 bis 9 zur Steigerung der Hautfeuchtigkeit.

13. Verwendung von Zubereitungen nach einem der Ansprüche 1 bis 9 zum Schutz der Haut vor Feuchtigkeitsverlust.

14. Verwendung von Zubereitungen nach einem der Ansprüche 1 bis 9 zur Steigerung der Tiefenfeuchtigkeit der Haut.

15. Verwendung von Zubereitungen nach einem der Ansprüche 1 bis 9 zur Verbesserung der Regeneration und der Reparatur ("Repair") der Haut nach einer UV-Bestrahlung und/oder einem Sonnenbad.

16. Verwendung von Zubereitungen nach einem der Ansprüche 1 bis 9 zum Schutz der Haut vor vorzeitiger Alterung und/oder langfristig auftretenden Hautschäden.

17. Verwendung von Zubereitungen nach einem der Ansprüche 1 bis 9 zur Verbesserung des Sonnenbrandschutzes der Haut (ermittelt gemäß COLIPA in vivo Methode).

18. Verwendung von Zubereitungen nach einem der Ansprüche 1 bis 9 zur Verhinderung und/oder Verminderung von Zell- und Hautschäden nach UV-Bestrahlung.

19. Verwendung von Zubereitungen nach einem der Ansprüche 1 bis 9 zur Verbesserung und/oder Steigerung des Eigenschutzes von Hautzellen und der Vitalität, der "Cellperformance" und des "Cellular Firmings".

**20.** Verwendung von Zubereitungen nach einem der Ansprüche 1 bis 9 zum Schutz und zur Verbesserung und/oder Steigerung des Immunschutzes der Haut und des Körpers.

**21.** Verwendung von Zubereitungen nach einem der Ansprüche 1 bis 9 zum Schutz der Haut vor UV und/oder lichtinduzierten irritativen und/oder allergischen Hautreaktionen, wie z. B. Sonnenallergie oder polymorphe Lichtdermatose.

**22.** Verwendung von Zubereitungen nach einem der Ansprüche 1 bis 9 zum Schutz der Haut vor optisch wahrnehmbaren Veränderungen.

**23.** Verwendung von Zubereitungen nach einem der Ansprüche 1 bis 9 zum unmittelbaren, sofort nach topischer Applikation auftretenden UV-Schutz der Haut.

**24.** Verwendung von Zubereitungen nach einem der Ansprüche 1 bis 9 für einen sich in der Haut aufbauenden, endogenen UV-Schutz, insbesondere des endogenen UVA-Schutzes.

**25.** Verwendung von Zubereitungen nach einem der Ansprüche 1 bis 9 zur Erreichung eines natürlichen Hautfarbtons bei und/oder nach Bestrahlung mit Sonnen- bzw. UV-Licht.

**26.** Verwendung von Zubereitungen nach einem der Ansprüche 1 bis 9 als Lichtschutzzubereitung.

Europäisches Patentamt

# EUROPÄISCHER RECHERCHENBERICHT

Nummer der Anmeldung
EP 05 10 0879

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DE 101 13 058 A1 (BASF AG) 19. September 2002 (2002-09-19) * Beispiele 1-13 * ----- | 1-26 | A61K7/42 |
| X | WO 03/053395 A (BEIERSDORF AG; GOEPPEL, ANJA; SCHULZ, JENS; KLETTE, ECKHARD) 3. Juli 2003 (2003-07-03) * Beispiele 1-7 * ----- | 1-26 | |
| P,X | US 2004/191191 A1 (EHLIS THOMAS [DE] ET AL) 30. September 2004 (2004-09-30) * Absatz [0196]; Beispiele 8a-30 * ----- | 1-26 | |
| X | EP 1 310 239 A (BEIERSDORF AG) 14. Mai 2003 (2003-05-14) * Beispiele 1-5 * ----- | 1-26 | |
| X | EP 1 352 639 A (BEIERSDORF AKTIENGESELLSCHAFT) 15. Oktober 2003 (2003-10-15) * Beispiele 1-5 * ----- | 1-26 | RECHERCHIERTE SACHGEBIETE (Int.Cl.7) |
| X | DE 102 14 054 A1 (BEIERSDORF AG) 9. Oktober 2003 (2003-10-09) * Beispiele 1-10 * ----- | 1-26 | A61K |
| X | WO 03/070201 A (BEIERSDORF AG; SCHULZ, JENS; GOEPPEL, ANJA; BUDOW, SIMONE) 28. August 2003 (2003-08-28) * Beispiele 1a-21 * ----- | 1-26 | |
| P,X | DE 103 27 468 A1 (BEIERSDORF AG) 5. Januar 2005 (2005-01-05) * Beispiele 1-6 * ----- | 1-26 | |

Der vorliegende Recherchenbericht wurde für alle Patentansprüche erstellt

1

| Recherchenort | Abschlußdatum der Recherche | Prüfer |
|---|---|---|
| München | 13. Juli 2005 | Yon, J-M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 05 10 0879

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-07-2005

| Im Recherchenbericht angeführtes Patentdokument | Datum der Veröffentlichung | Mitglied(er) der Patentfamilie | Datum der Veröffentlichung |
|---|---|---|---|
| DE 10113058 A1 | 19-09-2002 | AU 2461302 A | 19-09-2002 |
| | | BR 0200839 A | 25-03-2003 |
| | | CN 1382433 A | 04-12-2002 |
| | | EP 1240894 A2 | 18-09-2002 |
| | | JP 2002308751 A | 23-10-2002 |
| | | US 2002192167 A1 | 19-12-2002 |
| WO 03053395 A | 03-07-2003 | DE 10162840 A1 | 03-07-2003 |
| | | WO 03053395 A1 | 03-07-2003 |
| | | EP 1458345 A1 | 22-09-2004 |
| | | JP 2005513094 T | 12-05-2005 |
| | | US 2005048009 A1 | 03-03-2005 |
| US 2004191191 A1 | 30-09-2004 | WO 2004085412 A2 | 07-10-2004 |
| EP 1310239 A | 14-05-2003 | DE 10155957 A1 | 22-05-2003 |
| | | EP 1310239 A1 | 14-05-2003 |
| EP 1352639 A | 15-10-2003 | DE 10216508 A1 | 23-10-2003 |
| | | EP 1352639 A1 | 15-10-2003 |
| DE 10214054 A1 | 09-10-2003 | KEINE | |
| WO 03070201 A | 28-08-2003 | DE 10206798 A1 | 28-08-2003 |
| | | WO 03070201 A1 | 28-08-2003 |
| | | EP 1478324 A1 | 24-11-2004 |
| DE 10327468 A1 | 05-01-2005 | KEINE | |

EPO FORM P0461

Für nähere Einzelheiten zu diesem Anhang : siehe Amtsblatt des Europäischen Patentamts, Nr.12/82